# NEW EUROPEAN PATENT SPECIFICATION

(11) **EP 1 454 534 B2**
(45) Date of publication and mention of the opposition decision: **12.08.2020**
(45) Mention of the grant of the patent: 13.01.2010
(21) Application number: 02788615.9
(22) Date of filing: 15.11.2002
(51) Int. Cl.: A23P 10/30, A23L 27/00, A23G 4/06, C12N 1/16, B01J 13/02, A23K 10/00

(54) **MICROCAPSULES AND ORAL COMPOSITIONS CONTAINING THE SAME**
MIKROKAPSELN UND DIESE ENTHALTENDE ORALPRÄPARATE
MICROCAPSULES ET COMPOSITIONS POUR ADMINISTRATION PAR VOIE ORALE CONTENANT CES MICROCAPSULES

(30) Priority: 15.11.2001 JP 2001350836
(43) Date of publication of application: 08.09.2004
(73) Proprietor: SAN-EI GEN F.F.I., INC., Toyonaka-shi, Osaka 561-0828 (JP)
(72) Inventor: SASAKI, Yasushi, c/o SAN-EI GEN F.F.I., INC., Toyonaka-shi, Osaka 561-8588 (JP); ORIKOSHI, Hideyuki, c/o SAN-EI GEN F.F.I., INC., Toyonaka-shi, Osaka 561-8588 (JP); HAYASHI, Hideo, c/o SAN-EI GEN F.F.I., INC., Toyonaka-shi, Osaka 561-8588 (JP); MAEDA, Yoshiaki, c/o SAN-EI GEN F.F.I., INC., Toyonaka-shi, Osaka 561-8588 (JP)
(74) Representative: Müller-Boré & Partner Patentanwälte PartG mbB
(86) International application number: PCT/JP2002/011940
(87) International publication number: WO 2003/041509

(56) References cited:
- EP-A- 0 217 109
- EP-A- 0 242 135
- EP-A- 0 453 316
- EP-A- 1 161 878
- EP-A1- 0 528 466
- EP-A2- 0 460 945
- WO-A1-00/16643
- WO-A1-96/36433
- WO-A2-00/69440
- GB-A- 554 010
- JP-A- 5 253 464
- JP-A- 7 289 885
- JP-A- 8 243 378
- JP-A- 10 076 155
- JP-A- 2000 342 186
- US-A- 5 288 632
- US-A- 5 948 453
- PATENT ABSTRACTS OF JAPAN vol. 1997, no. 07, 31 July 1997 (1997-07-31) & JP 09 067205 A (SUMITOMO CHEM CO LTD; MITSUBISHI PAPER MILLS LTD), 11 March 1997 (1997-03-11)
- PATENT ABSTRACTS OF JAPAN vol. 2002, no. 06, 4 June 2002 (2002-06-04) & JP 2002 038133 A (KIRIN BREWERY CO LTD), 6 February 2002 (2002-02-06)
- PATENT ABSTRACTS OF JAPAN vol. 012, no. 186 (C-500), 31 May 1988 (1988-05-31) & JP 62 294079 A (MITSUBISHI PAPER MILLS LTD), 21 December 1987 (1987-12-21)
- DATABASE WPI Section Ch, Week 200135 Derwent Publications Ltd., London, GB; Class B04, AN 2001-334310 XP002359044 & RU 2 164 765 C1 (AFANASEV D S) 10 April 2001 (2001-04-10)
- DATABASE WPI Section Ch, Week 200376 Derwent Publications Ltd., London, GB; Class B04, AN 2003-809184 XP002359045 & KR 2002 042 297 A (NONG SHIM CO LTD) 5 June 2002 (2002-06-05)
- NAOTAKE ISHIWAKI: 'Kobo microcapsule-ka gijutsu no kaihatsu to oyo' THE FOOD INDUSTRY vol. 41, no. 18, 1998, pages 51 - 58, XP002963456
- JACSON L.S. ET AL. MICROENCAPSULATION AND THE FOOD INDUSTRY, LEBENSMITTEL-WISSENSCHAFT & TECHNOLOGIE vol. 24, no. 4, 1991, pages 289 - 297, XP001009807

## Description

The present invention relates to microcapsules of foreign substances encapsulated inside yeast cells. More specifically, the invention relates to microcapsules of foreign substances encapsulated inside yeast cells, wherein the speed, intensity and persistence of the release of foreign substances can be controlled. The present invention further relates to a manufacturing method for these microcapsules and to an oral composition containing the same.

So-called microencapsulation methods known in the art involve enclosing a desired component inside particle containers (microcapsules) made of natural or synthetic macromolecules and having a diameter of from a few to several hundred µm in order to control the release speed and the release times thereof so as to increase its persistence. Well-known microencapsulation methods include coacervation methods using gelatin (e.g. US Patent Nos. 2800457, 2800458), in-situ methods involving the building of an outer (aqueous phase) membrane (e.g. Japanese Published Examined Application Nos. S36-9168, S47-23165, Japanese Published Unexamined Application Nos. S48-57892, S51-9079, S54-25277), and interfacial polymerization methods using a film-forming reaction between the inner and outer phases; in recent years microorganism microencapsulation methods wherein the cell membranes of microorganism such as yeast are used as capsule films have also become widely known.

Microcapsule manufacturing methods using microorganism cells include methods wherein flavorings or other desired foreign substances are encapsulated inside yeast cells without removing the contents of the cells (as disclosed for example in Japanese Published Unexamined Application Nos. S61-88871, S62-186937, S63-88033, S58-107189, and WO94/22572, WO96/36433, EP 0242135B1, etc.), as well as methods wherein the desired foreign substances are encapsulated in yeast cells after removing the endogenous intracellular components (as disclosed for example in Japanese Published Unexamined Application Nos. H4-4033, H4-63127, H4-117245, H5-15770, H8-243378, etc.). The latter method is advantageous in that the greater the amount of endogenous intracellular components that can be removed from the yeast cells, the greater the amount of foreign substances that can be encapsulated therein, thereby making it possible to increase the effect of these foreign substances.

In the food industry, for example, chewing gum is a kind of product that demands a rapid but sustained release of taste and aroma (flavor), which is difficult to achieve in practice, since although taste and flavor are brought forth intensely upon initial chewing, it tends to become rapidly weaker as chewing proceeds. Therefore, methods allowing the flavor to be sustained as long as possible have been actively studied. Japanese Published Unexamined Application No H5-192085 proposes a method for preparing gum using a flavoring agent obtained by enclosing a flavoring inside a microorganism cell thereby making it possible to obtain a chewing gum with a strong and sustained flavor, using a small amount of flavoring.

However, the release speed, intensity and persistence of flavorings or spices (sweeteners, etc.) as foreign substances (constituents) enclosed in microorganism cells are by no means yet sufficient and requires further improvement.

Further, EP-A-0 217 109 describes a chewing gum wherein the gummy mass is provided with a plurality of microcapsules dispersed therein, wherein each microcapsule contains active alimentary, confectionary of medical substances, and the microcapsules are made of materials suitable for releasing the substances contained therein after preset times. Furthermore, Jacson et al. (Lebensmittel-Wissenschaft und Technologie, vol. 24, no. 4, pages 289-297, 1991) describes the use of microencapsulation in food industry, WO-A-00/16643 describes encapsulated aromas and/or odorous substances which are covered with modified cellulose, wherein said cellulose presents a reversible gel formation at increased temperatures, EP-A-0 528 466 describes chewing gum containing a flavor encapsulated in microorganism cells, and EP-A-0 453 316 describes a process for the preparation of microcapsules comprising the step of eluting the intracellular components of a yeast to the outside of its cells and the step of making a hydrophobic liquid enclosed in the yeast cells from which the intracellular components have been eluted.

The purpose of the present invention is to provide microcapsules of foreign substances encapsulated inside yeast cells, and also to improve the release properties of these encapsulated foreign substances (constituents), increasing the speed, intensity and persistence of the release of constituents. A further purpose of the present invention is to provide an oral composition wherein the effect of such encapsulated foreign substances can be achieved more efficiently with smaller amounts of foreign substances, or an oral composition having an excellent persistence of the encapsulated foreign substances with smaller amounts of foreign substances, by using the aforementioned microcapsules.

As a result of a diligent study towards solving the above problems, the present inventors discovered that in so-called microorganism microcapsules of foreign substances encapsulated inside yeast cells, by partially or completely covering these yeast cells (capsules) with at least one member selected from the group consisting of saccharides and proteins, it is possible to improve the speed, intensity and persistence of the release of these foreign substances while preventing their degradation. The inventors also noticed that these effects are notably improved by using yeast cells whose endogenous intracellular component has been previously removed for encapsulating foreign substances. The present invention is achieved based on these findings.

Specifically, the present invention relates to the embodiments as characterized in the appended claims.

### (1) Microcapsules

The microcapsules according to the present invention are as defined in claims 1 to 7.

Microorganism used in the present invention for encapsulating foreign substances are yeasts. Yeasts that may be used include yeasts that can be properly ingested by the human body such as brewer's yeasts, baker's yeasts, torula yeasts, etc. Specific examples of yeasts include for example yeasts belonging to *Saccharomyces* such as *Saccharomyces cerevisiae, Saccharomyces rouxii, Saccharomyces carlsbergensis,* etc. and Candida yeasts such as *Candida utilis, Candida tropicalis, Candida lipolytica, Candida flaveri,* etc. Such yeast cells may be used singly or in combination of 2 or more. These yeast cells are not restricted in any particular way, but their particle size ranges preferably from 1µm to 20µm.

These yeast cells may be used in any state for encapsulating the foreign substances, as viable or dead yeast cells, in a wet or dry state, and with or without their endogenous intracellular component removed by elution. Yeast cells with their endogenous intracellular components removed (including removed by elution) beforehand are preferably used. Endogenous intracellular components herein include amino acids, peptides, proteins (including enzymes), sugars, nucleic acids, fats/lipids, etc. Elution of endogenous intracellular components allows herein enclosing more foreign substances inside the yeast cells while significantly suppressing undesirable smells and tastes elicited by the endogenous intracellular components and also preventing any possible decomposition or denaturation of the foreign substances brought about by the endogenous intracellular component.

The method for eluting the intracellular components (endogenous content) is not particularly restricted and may include methods known in the art or methods according to future developments. Conventional methods include for example physical treatment methods involving thermal treatment, pH treatment and cell wall crushing; chemical methods involving the addition of elution promoting agents; enzymatic methods using enzymes for dissolving out the intracellular component or enzymes for cell-wall lysis, and combinations of these methods (Japanese Published Unexamined Application No. H4-4033).

Thermal treatment herein can be carried out by heating a suspension of the target yeast cells usually at 30 to 100°C, preferably at 30 to 60°C, while stirring for several minutes to several hours. Elution promoting agents may be simultaneously used in order to increase the efficiency of the elution of the intracellular component. Suitable elution promoting agents include for example polar organic solvents such as ethanol, propanol and other lower alcohols, ethyl acetate, acetone, and other polar organic solvents; inorganic bases, sugars, quaternary ammonium salts, various kinds of fungicides, bactericides, germicides, and bases such as sodium hydroxide, potassium hydroxide, etc. A specific example of such a method involves adding a solvent such as acetone or the like to an aqueous dispersion of the yeast and then shaking at about 40°C for about 24 hours.

In cell-wall crushing treatments, the breaking of the cell walls may be accomplished using a sonicator, a mill, etc. A specific example of such a method involves treating an aqueous dispersion of the yeast for 10 minutes in a bead mill.

Enzymatic methods using enzymes for dissolving out the intracellular components may involve the use of autolytic enzymes from the yeast itself (Babayan, T.L. and Bezrukov, M.G., 1 Acta Biotechnol. 0,5,129-136 (1985)), and enzymatic treatment methods with protease alone, or protease in combination with at least one selected from nuclease, β-glucanase, esterase and lipase. A concrete way of implementing such a method may involve for example incubating for 1 to 48 hours at 30 to 60°C an aqueous dispersion of the yeast possessing autolytic enzymes, or an aqueous dispersion of a microorganism to which the aforementioned enzymes have been added.

Enzymatic treatments for cell-wall lysis involve treating the yeast cells using for example at least one type of enzyme such as glucanase (β-1, 3-glucanase), mannanase, chitinase, etc. that decomposes the constituents of the yeast's cell wall (polysaccharides such as glucan and mannan, complexes of these polysaccharides and proteins, chitin, etc.). A concrete way of implementing such an enzymatic treatment for cell-wall lysis may involve for example incubating an aqueous dispersion of the yeast to which has been added the aforementioned enzymes for several minutes to about 10 hours at 30 to 60°C, ordinarily at a pH from 4 to 9.

The material thus obtained may be further centrifuged, discarded the supernatant, while the residue (cells) is subsequently washed, heated and/or pH adjusted as needed, in order to obtain yeast cells (variants) with their intracellular component removed.

In those yeast cells with their endogenous intracellular components eluted used in the present invention, the elution method and the extent of elution are not restricted to the aforementioned examples, but is meant to include a wide range of other methods wherein the intracellular constituents are also removed by elution. Yeast cells with their endogenous cell components eluted include herein preferably yeast cells wherein the proportion of absolute dry weight of eluted component (elution rate) relative to 100 wt% of absolute dry weight of untreated yeast cells ranges from 10 to 80 wt%, and more preferably from 30 to 70 wt%.

Commercially available products may be conveniently used as yeast cells with their endogenous intracellular components (endogenous components) removed. Such products include for example, but are not restricted to, commercially available yeast cell walls from Oriental Yeast, Co., Ltd.; Tanabe Seiyaku Co. Ltd.; Asahi Food & Healthcare Ltd.; Kirin Brewery Co. Ltd., etc. (all Japanese firms).

In order to encapsulate as much foreign substance as possible inside these yeast cells (cell residues) having had their endogenous intracellular component removed, further treatments may be performed as needed in addition to those listed above, for example acid treatments (Japanese Published Unexamined Application No. H8-243378), alkali treatments (Japanese Published Examined Application No. H7-32871), alcohol treatments (Japanese Published Examined Application No. H8-29246), etc. Such aforementioned methods known in the art are not restricted, being limited only by the allowable physics and chemistry of the prepared yeast cell walls acting as microcapsule films.

In an acid treatment, specifically, the cell residues obtained by enzymatic treatment or by other methods above are suspended for example in an acidic aqueous solution (preferred pH 2 or less) of an inorganic acid such as hydrochloric acid, phosphoric acid or sulfuric acid, or an organic acid such as lactic acid, citric acid, acetic acid or an ascorbic acid, and then heated (preferably at 50 to 100°C) while stirring for a certain time.

An alkali treatment may be carried out for example by stirring the cell residues obtained by enzymatic treatment or by other methods as above for several minutes to several hours in an alkaline aqueous solution having preferably a pH of 9 to 13, and more preferably from 10 to 12. The temperature of this aqueous solution is not restricted in any particular way but ranges ordinarily from 20 to 100°C, preferably from 30 to 100°C, and yet more preferably from 50 to 80°C. Compounds used for preparing the alkaline aqueous solution include for example aqueous solutions of inorganic salts such as sodium hydroxide, calcium hydroxide, potassium hydroxide, sodium silicate, etc.; and organic nitrogen compounds such as ammonia, monoethanoldiamine, ethylenediamine, diethylenetriamine, etc.

In an alcohol treatment, for example, alcohols such as monohydric alcohols, etc., or hydroalcohols may be added to the cell residues obtained by enzymatic treatment or by other methods as above and stirred for several minutes to several hours. The temperature of the treatment solution is not particularly restricted provided the alcohol does not evaporate, and ranges ordinarily from 20 to 80°C. The temperature ranges herein preferably from 30 to 60°C. More preferred methods involve for example stirring while heating at 40 to 50°C. Monohydric alcohols include for example lower alcohols having 1 to 6 carbon atoms such as methanol, ethanol, propanol, isopropyl alcohol, butanol, hexanol, etc., preferably ethanol.

The foreign substance encapsulated in the yeast cell is liquid. Examples thereof include, but not limited to, a hydrophilic, hydrophobic or an amphiphilic liquid. The foreign substance is preferably a hydrophobic substance. A foreign substance herein refers to a substance artificially added to the yeast cell to be encapsulated therein. This includes for example substances not derived from the yeast components (endogenous components) as well as substances derived from the yeast, substances extracted from the yeast and then concentrated, mixed with other components or subjected to other kinds of artificial treatment. Placing these substances back into the cell counts herein as an artificial treatment; these substances are thus regarded as foreign substances according to the present invention.

Examples of the former include for example food additives, food compositions, pharmaceutical compositions, pharmaceutical additives, quasi-pharmaceutical compositions, cosmetic compositions, etc. Examples of the latter include for example microbial extracts such as yeast extracts, or bioactive substances derived from (or produced by) microorganism cells such as antibiotics or immunosuppressive pharmaceuticals, etc. Preferred herein are edible compositions such as food additives, food compositions (including food raw materials), oral pharmaceutical compositions, and oral pharmaceutical additives.

Specific examples of foreign substances include for example flavorings (including flavor components), colorings (including colorants, pigments and dyestuffs), sweeteners (including saccharides), acidulants, seasonings, bitterants, spices, fats/lipids, fatty acids, amino acids, enzymes, vitamins, animal or plant extracts, bioactive substances, etc. Preferred herein are components that can form flavor or taste (flavor/taste components) such as flavorings, sweeteners, acidulants, seasonings, bitterants, spices, fats/lipids, amino acids, vitamins, or, animal or plant extracts, etc.; colorings, vitamins, bioactive substances, etc. Yet more preferred herein are flavor/taste components, in particular flavorings, sweeteners, acidulants, seasonings, bitterants and spices.

These foreign substances may be foreign substances (active ingredients) by themselves having their intrinsic hydrophobic, hydrophilic or amphoteric character, preferably hydrophobic, or preparations wherein the foreign substances are heated or mixed with other substances such as solvents, preferably hydrophobic preparations.

Flavorings include herein ordinary flavorings widely used in the food, pharmaceutical and cosmetic industries, preferably flavorings suitable to be used in edible products such as foods, etc. Specific examples include for example citrus flavors such as orange, lemon, lime, grapefruit, mandarin, tangerine, etc.; other fruit flavors such as apple, banana, cherry, grape, melon, peach, pineapple, plum, raspberry, strawberry, etc.; bean flavors such as vanilla, coffee, cocoa, chocolate, etc.; mint flavors such as peppermint, spearmint etc.; spice flavors such as allspice, cinnamon, nutmeg, etc., nut flavors such as almond, peanut, walnut, etc.; aquatic product flavors such as crab, shrimp, fish, shellfish, etc.; and various kinds of other flavors from vegetables, cereals, seaweeds, etc. Flavor components include for example menthol, d1-menthol, menthone, vanillin, ethyl vanillin, cinnamic acid piperonal, d-borneol, maltol, ethyl maltol, camphor, methyl anthranilate, methyl cinnamate, cinnamic alcohol, methyl-N-methyl anthranilate, methyl β-naphthyl ketone, limonene, linalool, allyl isothiocyanate, etc. These may be used singly or in combinations of 2 or more.

Natural colors and synthetic colors may be used as colorings. Preferred herein are colorings suitable to be used in edible products such as food, etc. Examples of natural colors include carotenoid-based colors such as carotenoid colors, paprika color, annatto color, orange color, tomato color, marigold color, etc.; anthocyanin colors such as purple sweet potato color, red cabbage color, elderberry color, grape juice color, grape skin color, purple corn color, red radish color, perilla color, red kernel rice color, cowberry color, gooseberry color, cranberry color, salmonberry color, thimbleberry color, strawberry color, dark sweet cherry color, cherry color, hibiscus color, huckleberry color, black currant color, blackberry color, blueberry color, plum color, whortleberry color, boysenberry color, mulberry color, purple yam color, raspberry color, red currant color, loganberry color, etc.; quinoid-based colors such as cochineal color, Shikon color, madder color, Lac color, etc.; flavonoid-based colors such as cacao color, kooroo color, kaoliang color, Sandalwood red color, onion color, tamarind color, persimmon color, carob germ color, licorice color, Sappan wood color, peanut color, pecan nut color, Carthamus red color, Carthamus yellow color, etc.; azaphilone-based colors such as Monascus color, Monascus red color, etc.; and other colors such as Monascus yellow color, caramel, turmenic color, Kusagi color, Gardenia blue color; Gardenia yellow color, Gardenia red color, chlorophyllin color, chlorophyll, spirulina blue color, etc. Synthetic coal tar colors include for example Food Red No.2 (Amaranth), Food Red No.3 (Erythrosine), Food Red No.40 (Allura Red AC), Food Red No.102 (New Coccine), Food Red No.104 (Phloxine B), Food Red No.105 (Rose Bengale), Food Red No.106 (Acid Red), Food Yellow No.4 (Tartrazine), Food Yellow No.5 (Sunset Yellow FCF), Food Blue No.1 (Brilliant Blue FCF), Food Blue No.2 (Indigo Carmine), Food Green No.3 (Fast Green FCF), etc. Natural color derivatives include for example sodium norbixinate, potassium norbixinate, copper chlorophyll, sodium copper chlorophyllin and sodium iron chlorophyllin, etc.; synthetic natural colors include for example β-carotene, lutein, astaxanthin, canthaxanthin, riboflavin, riboflavin acetate, 5'-riboflavin phosphate ester sodium, etc. Preferred herein are β-carotene, carotenoid colors, paprika color, annatto color, madder color, orange color, Gardenia colors (Gardenia blue color; Gardenia yellow color, Gardenia red color), chlorophyll, Shikon color, Food Red No.3 (Erythrosine), Food Yellow No.4 (Tartrazine), onion color, tomato color, marigold color, lutein color, etc.

Sweeteners (including saccharides) include herein for example monosaccharides, disaccharides, oligosaccharides, sugar alcohols and high-intensity sweeteners. Specific examples thereof include for example monosaccharides such as arabinose, galactose, xylose, glucose, sorbose, fructose, rhamnose, ribose, isomerized liquid sugar (high fructose corn syrup), N-acetyl glucosamine, etc.; disaccharides such as isotrehalose, sucrose, trehalulose, trehalose, neotrehalose, palatinose, maltose, melibiose, lactulose, lactose, etc.; oligosaccharides such as α-cyclodextrin, β-cyclodextrin, isomalto oligosaccharides (isomaltose, isomaltotriose, panose, etc.), oligo-N-acetyl glucosamine, galactosyl sucrose, galactosyl lactose, galactopyranosyl (β1-3) galactopyranosyl (β1-4) glucopyranose, galactopyranosyl (β1-3) glucopyranose, galactopyranosyl (β1-6) galactopyranosyl (β1-4) glucopyranose, galactopyranosyl (β1-6) glucopyranose, xylooligosaccharides (xylotriose, xylobiose, etc.), gentioligosaccharides (gentiobiose, gentiotriose, gentiotetraose, etc.), stachyose, Theander oligosaccharides, nigero-oligosaccharides (nigerose, etc.), palatinose oligosaccharides, palatinose syrups, fucose, fructooligosaccharides (kestose, nistose, etc.), fructofuranosyl nistose, polydextrose, maltosyl β-cyclodextrin, maltooligosaccharides (maltotriose, tetraose, pentaose, hexaose, heptaose, etc.), raffinose, glycosyl sucrose syrups (coupling sugars), soybean oligosaccharides, invert sugars, glucose syrups, etc.; sugar alcohols such as isomaltitol, erythritol, xylitol, glycerol, sorbitol, palatinit, maltitol, maltoteraitol, maltotriitol, mannitol, lactitol, reduced isomalto-oligosaccharides, reduced xylooligosaccharides, reduced gentioligosaccharides, reduced maltose syrup, reduced glucose syrups, etc.; and high-intensity sweeteners such as α-glucosyltransferase treated stevia, aspartame, acesulfame potassium, alitame, liquorice extract (glycyrrhizin), triammonium glycyrrhizate, tripotassium glycyrrhizate, trisodium glycyrrhizate, diammonium glycyrrhizate, dipotassium glycyrrhizate, disodium glycyrrhizate, curculin, saccharine, saccharine sodium, cyclamate, sucralose, stevia extract, stevia powder, dulcin, thaumatin (somatin), tenryocha (Chinese blackberry tea) extract, serendipity berry extract, neotame, neohesperidine dihydrochalcone, fructosyltransferase treated stevia, Brazilian licorice extract, miracle fruit extract, luohan fruit extract, enzyme-treated liquorice, enzyme-decomposed liquorice, etc., and others sweeteners such as honey, fruit juices fruit juice concentrates, etc.

Fats/lipids include herein for example soybean oil, corn oil, rice bran oil, sunflower oil, cottonseed oil, olive oil, castor oil, fish oil, lard, beef tallow, sheep tallow, horse fat, lecithin, etc.

Fatty acids include for example docosahexaenoic acid, eicosapentaenoic acid, linoleic acid, linolenic acid, palmitic acid, oleic acid, etc.

Vitamins include for example vitamin A, B-group vitamins (vitamins B₁, B₂, B₆, B₁₂, B₁₃, B_{c}, Bₜ, etc.), vitamin C, vitamin D, vitamin E, vitamin H, vitamin K, vitamin M, vitamin P, vitamin U, etc., and their derivatives.

Animal or vegetable extracts include herein ordinary animal or vegetable extracts widely used in the food, pharmaceutical and cosmetic industries, preferably those suitable for use in edible products such as foods, etc. Examples thereof include for example vegetable extracts such as wheat extract, vanilla extract, quillaja extract, aloe vera extract, amacha extract, wheat germ extract, udo extract, gum benzoin extract, Aloe arborescens extract, konjac extract, eucalyptus leaf extract, cinchona extract, sour orange extract, Amur cork tree extract, Gentiana extract, grape seed extract, tea extract, etc.; and animal extracts such as beef extract, chicken extract, pork extract, crab extract, bonito extract, oyster extract, gelatin, collagen, yeast extract, etc. Animal or vegetable extracts include herein extracts of processed animals or vegetables such as extracts of dried shaved bonito flakes, etc., and animal or vegetable extracts subjected to further processing such as gelatin hydrolysates.

Bioactive substances include herein ordinary bioactive substances widely used in the food, pharmaceutical and cosmetic industries, preferably those suitable for use in edible products such as foods, oral pharmaceuticals, etc. Examples thereof include for example ethenzamide, sodium salicylate, acetoaminophen, ibuprofen, phenacetin, eugenol, antipyrine, catechins, saponins, chondroitin sulfate, phospholipids such as ceramides, etc.

Spices include herein ordinary spices widely used in the food, pharmaceutical and cosmetic industries. Preferred herein are those suitable for use in edible products such as foods, oral pharmaceuticals, etc. Examples thereof include, but not limited to, garlic, onion, lemongrass, chervil, tarragon, laurel, rosemary, basil, chili pepper, thyme, cinnamon, cashew, oregano, turmeric, clove, pepper, coriander, etc.

The components in the aforementioned groups belonging to the same category (i.e. groups of colorings, sweeteners, etc.) may be used singly or in combination of 2 or more.

Each of the aforementioned foreign substance groups may also be used singly or in combination of 2 or more. To the aforementioned foreign substances may also be added dispersants, antioxidants, preservatives, flavor improvers, emulsifiers, reinforcing agents, etc. For example, at least one antioxidant selected from the group consisting of extracted tocopherol, dl-α-tocopherol, d-α-tocopherol, dl-γ-tocopherol, mixed tocopherol, rosemary extract, Japanese bayberry extract, rutin extract, enzymatic hydrolysates of rutin, tea extract and tocotrienol may be used simultaneously with to the foreign substances encapsulated in the yeast cells, in particular flavor/taste components, preferably flavorings.

The aforementioned foreign substances may be encapsulated inside the aforementioned yeast cells by mixing the yeast cells (cell bodies) with the foreign substances. Specifically, the foreign substances may be encapsulated inside the yeast cells by adding the foreign substance to a liquid dispersion of yeast cells (cell bodies), adjusting pH and temperature to desired values, and stirring as needed for a predetermined period of time. The pH is not particularly restricted herein and may be suitable selected ordinarily from within the range from 5 to 9, and preferably from 6 to 8. Temperature values are not particularly restricted and may be suitably selected so as to range ordinarily from 40 to 80°C, and preferably from 50 to 70°C.

There is no particular restriction as to the necessity of stirring, either. If any stirring equipment is used for stirring, for example blenders with stirring blades, emulsifying devices, dispersing devices, homogenizers, etc., the encapsulation of the foreign substance inside the yeast cells can be carried out more effectively. The stirring speed or stirring rotation number per minute is not particularly restricted herein and may be suitable selected so as to range usually from 1,000 to 10,000 rpm.

When mixing the foreign substance with the aforementioned yeast cells (cell bodies), other constituents may be further included in the mixture system of foreign substance and yeast cells, for example hardeners, antioxidants, stabilizers, dispersants, emulsifiers, pH adjustment agents, preservatives, anti-denaturation agents, etc.

Examples of saccharides include for example monosaccharides such as arabinose, galactose, xylose, glucose, sorbose, fructose, rhamnose, ribose, isomerized liquid sugar (high fructose corn syrup), N-acetyl glucosamine, etc.; disaccharides such as isotrehalose, sucrose, trehalulose, trehalose, neotrehalose, palatinose, maltose, melibiose, lactulose, lactose, etc.; trisaccharides such as maltotriose, etc.; tetrasaccharides such as maltotetraose, etc.; oligosaccharides and polysaccharides such as α-cyclodextrin, β-cyclodextrin, isomalto oligosaccharides (isomaltose, isomaltotriose, panose, etc.), oligo-N-acetyl glucosamine, galactosyl sucrose, galactosyl lactose, galactopyranosyl (β1-3) galactopyranosyl (β1-4) glucopyranose, galactopyranosyl (β1-3) glucopyranose, galactopyranosyl (β1-6) galactopyranosyl (β1-4) glucopyranose, galactopyranosyl (β1-6) glucopyranose, xylooligosaccharides (xylotriose, xylobiose, etc.), gentioligosaccharides (gentiobiose, gentiotriose, gentiotetraose, etc.), stachyose, theander oligosaccharides, nigero-oligosaccharides (nigerose, etc.), palatinose oligosaccharides, palatinose syrups, fucose, fructooligosaccharides (kestose, nistose, etc.), fructofuranosyl nistose, polydextrose, maltosyl β-cyclodextrin, maltooligosaccharides (maltotriose, tetraose, pentaose, hexaose, heptaose, etc.), raffinose, glycosyl sucrose syrups (coupling sugars), soybean oligosaccharides, invert sugars, glucose syrups, dextrin, gum arabic, xanthan gum, guar gum, carrageenan, curdlan, tamarind seed gum, gellan gum, starches, modified starches, etc.; sugar alcohols such as isomaltitol, erythritol, xylitol, glycerol, sorbitol, palatinit, maltitol, maltoteraitol, maltotriitol, mannitol, lactitol, reduced isomalto-oligosaccharides, reduced xylooligosaccharides, reduced gentioligosaccharides, reduced maltose syrup, reduced glucose syrups, etc. Preferred herein are monosaccharides such as glucose, ribose, arabinose, xylose, mannose, galactose, fructose, etc.; disaccharides such as maltose, lactose, sucrose, trehalose, etc.; trisaccharides such as maltotriose, etc.; tetrasaccharides such as maltotetraose, etc.; oligosaccharides such as maltooligosaccharides, etc.; polysaccharides such as dextrin, gum arabic, xanthan gum, guar gum, gellan gum, starches, modified starches, cyclodextrin, etc.; sugar alcohols such as palatinit, sorbit, xylit, maltitol, lactitol, erythritol. Yet more preferred are disaccharides such as lactose, trehalose, etc. Such saccharides may be used singly or in combination of 2 or more. The saccharides used according to the present invention are as defined in the claims.

Proteins include for example gelatin, gelatin hydrolysates, maize proteins, casein, sodium caseinate, collagen, etc. They include preferably gelatin, maize proteins and sodium caseinate. These proteins may be used singly or in combination of 2 or more.

Furthermore, these saccharides and proteins may also be used singly or in combination of 2 or more.

The methods for coating saccharides and proteins (hereinafter referred to as 'saccharides, etc.') onto the aforementioned microcapsules are not restricted in any particular way. They may include for example methods wherein the microcapsules (foreign-substance encapsulating yeast cells) prepared according to a method described above are dried in a spray-drying machine and are then sprayed using a fluidized bed granulator, etc. with solutions or dispersions of 'saccharides, etc.' dissolved or dispersed in water or other solvent; methods wherein a bed spread over with the 'saccharides, etc.' (fluid bed) is sprayed with a dispersion of microcapsules (foreign-substance encapsulating yeast cells), and then the droplets of microcapsules with their surface covered with 'saccharides, etc.' are taken out and dried; methods wherein the 'saccharides, etc.' are added to a liquid dispersion of the microcapsules (foreign-substance encapsulating yeast cells), are homogeneously dissolved therein and are then lyophilized; methods wherein the microcapsules (foreign-substance encapsulating yeast cells) are dried by spray-drying, etc. and the 'saccharides, etc.' are mixed therewith as a powder mixture using a high speed stirring mixer, etc.; and methods wherein the 'saccharides, etc.' are added to a liquid dispersion of the microcapsules (foreign-substance encapsulating yeast cells), are homogeneously dissolved therein and this liquid dispersion is then spray-dried, etc. Preferred methods are those wherein the 'saccharides, etc.' are added to a liquid dispersion of the microcapsules (foreign-substance encapsulating yeast cells), are homogeneously dissolved therein and this liquid dispersion is then spray-dried.

The proportion of the 'saccharides, etc.' (saccharides or proteins) relative to 100 wt% of the above microcapsules (foreign-substance encapsulating yeast cells) usually ranges from 1 to 90 wt%, preferably from 10 to 50 wt%, and yet more preferably from 20 to 40 wt% (dried solids weight ratios).

In the above method, if the microcapsules (foreign-substance encapsulating yeast cells) are used as a dispersion/suspension in a liquid solution, the proportion of microcapsules in these liquid dispersion/suspension usually ranges from 5 to 80 wt%, preferably from 10 to 50 wt%, yet more preferably from 20 to 40 wt%; if the 'saccharides, etc.' are also used as a liquid solution or dispersion/suspension, their proportion in these solutions usually ranges from 1 to 60 wt%, preferably from 10 to 50 wt%, and yet more preferably from 30 to 40 wt%.

The microcapsules thus obtained have 'saccharides, etc. (saccharides or proteins) coated at least partially onto their surfaces. Herein, the surface of the microcapsules is preferably caused to be coated partially or completely with 'saccharides, etc.'. The 'saccharides, etc.' may also permeate /penetrate into the microcapsule film (cell wall) or into the interior of the microcapsule, so long as some 'saccharides, etc.' remain coated at least partially to the surface of the microcapsule.

Such microcapsules according to the present invention, i.e. microcapsules with 'saccharides, etc.' coated to their surfaces or, preferably, having their surfaces covered with 'saccharides, etc.', may be used as components in foods, pharmaceuticals, quasi-pharmaceuticals, cosmetics and feeds. The microcapsules according to the present invention may be employed preferably as components in oral compositions used orally (food compositions, oral pharmaceutical or quasi-pharmaceuticals compositions, and feed compositions). Oral compositions include herein physiologically ingestible edible compositions as well as buccal compositions, edible or not, for use in the mouth.

The microcapsules according to the present invention (microcapsules with coated 'saccharides, etc.') may have different applications depending on the kind of the encapsulated components. For example, microcapsules enclosing flavorings may be prepared and used as flavorings (microencapsulated flavorings); microcapsules enclosing sweeteners, as sweeteners (microencapsulated sweeteners); microcapsules enclosing seasonings, as seasonings (microencapsulated seasonings); microcapsules enclosing acidulants, as acidulants (microencapsulated acidulants); microcapsules enclosing bitterants, as bitterants (microencapsulated bitterants); microcapsules enclosing spices, as spices (microencapsulated spices); and microcapsules enclosing colorings, as colorants (microencapsulated colorants).

The microcapsules according to the present invention may be suitably used as a component in embodiments of oral compositions that are ingested while being held or chewed in the mouth. Specific examples of such oral compositions include for example chewing gum, soft candy such as gummy, nougat, etc., or food compositions held in the mouth for a prolonged time such as sweets, nonessential grocery foods etc.; pharmaceutical or quasi-pharmaceutical compositions that are ingested while being held or chewed in the mouth such as chewable tablets, masticatories, granules, troche tablets, buccal tablets, dentifrices, etc. Preferred herein are compositions ingested while being chewed for a prolonged time in the mouth such as chewing gum. In the present invention, the term chewing gum applies in a broad sense to any gums taken by chewing in the mouth including bubble gum, etc.

The present invention provides oral compositions containing the aforementioned microcapsules with coated 'saccharides, etc.' (microcapsules with coated 'saccharides, etc.': microcapsules according to the present invention). As follows from all the above, the oral compositions according to the present invention encompass thus compositions used in the mouth (buccal compositions) and compositions ingested (eaten) orally (edible compositions), such as food compositions, oral pharmaceuticals or quasi-pharmaceuticals compositions, and feed compositions.

Specific examples of such oral compositions are similar to those listed above, for example the aforementioned chewing gum, soft candy such as gummy, nougat, etc.; food compositions held in the mouth for a prolonged time such as sweets, nonessential grocery foods, etc.; pharmaceutical or quasi-pharmaceutical compositions that are taken while being held or chewed in the mouth for prolong time such as chewable tablets, masticatories, granules, troche tablets, buccal tablets, dentifrices, etc. Preferred herein are food compositions, and more preferably chewing gum.

Apart from containing as constituents with microcapsules with coated 'saccharides, etc.' (microcapsules according to the present invention), the oral compositions according to the present invention, especially food compositions and in particular chewing gum may also contain constituents of conventional oral compositions, especially of food compositions and in particular of chewing gum, and may be manufactured using conventional methods.

In the case of a chewing gum, there is no restriction as to the amount of microcapsules according to the present invention (microcapsules with coated 'saccharides, etc.') in 100 wt% of the final chewing gum, but it usually ranges from 0.05 to 10 wt%, preferably from 0.1 to 5 wt%, and yet more preferably from 0.5 to 3 wt%.

The microcapsules according to the present invention in oral compositions such as chewing gum, etc. are not particularly restricted concerning their intended use, but they contain inside preferably at least one of the following: flavorings, sweeteners (including saccharides), colorings, acidulants, vitamins, spices, animal/plant extracts, bioactive substances, seasonings, bitterants, fats/lipids, fatty acids, amino acids or enzymes. Preferred in case of a chewing gum are components for building flavor or taste (flavor/taste components), in particular flavorings, sweeteners, acidulants, seasonings or bitterants. Microcapsules (oral compositions) according to the present invention containing these flavorings, sweeteners, acidulants, seasonings or bitterants may be formulated respectively in a chewing gum (oral composition) as, respectively, microencapsulated flavorings, microencapsulated sweeteners, microencapsulated acidulants, microencapsulated seasonings and microencapsulated bitterants.

Specific examples of such foreign substance components (flavorings, sweeteners, colorings, etc.) include the same examples as listed above, but are not limited thereto. For example, flavorings other than the flavorings listed above may also be used as flavor component.

Apart from the microcapsules according to the present invention, the chewing gum formulation may include other components such as gum base, sweeteners (including saccharides), colorings, thickeners, acidulants, softeners, etc. The amount of gum base in the chewing gum ranges ordinarily from 10 to 35 wt%, and preferably from 20 to 30 wt%. Sweeteners herein include for example saccharides such as sucrose, fructose, liquid sugar, glucose, oligosaccharides, etc.; high-intensity sweeteners such as aspartame, sucralose, acesulfame potassium, somatin, stevia, alitame, neotame, xylitol, saccharine salts, glycyrrhizin, etc.

Chewing gums may be prepared for example in accordance with the methods and procedures described in "Gum Base and Gum Products Technology" and "The Great American Chewing Gum Book" by Robert Hendrickson, published by CAFOSA Gum S/A (1974). The constituent components of the microcapsules according to the present invention may be incorporated therein in any arbitrary process step, but preferably close to the end of the manufacturing process, especially in such cases where the foreign substances to be encapsulated in the microcapsules are sensitive to heat, for example flavor/taste components (flavorings in particular), colorings, etc., in order to prevent the thermal alternation of these foreign substances (i.e. loss or denaturation of flavor or color, etc.).

### (2) Manufacturing method for microcapsules

The present invention relates further to a manufacturing method as defined in claims 8 to 10.

Between steps 1) and 2) the yeast cells encapsulating the foreign substance may be subject as needed to further treatments, such as separation, washing, dehydration, drying, etc.

Alternatively, yeast cells with their endogenous intracellular component eluted beforehand may be used as the yeast cells of step 1) above.

The yeast cells, foreign substances, saccharides and proteins used in the aforementioned manufacturing method may be those described in section (1) above, while the methods for eluting the endogenous intracellular component out of the yeast cells, the methods for encapsulating the foreign substances in the microorganism cells, and the methods for coating saccharides and proteins onto the surface of the yeast may be carried out also in accordance with the method described in section (1) above.

### (3) Method for controlling the expression of flavor/taste or the release of flavor/taste components

The present invention also relates to a method for controlling the speed, intensity and persistence of the release of foreign substances in microcapsules of foreign substances encapsulated in yeast cells (foreign-substance encapsulating yeast cells), preferably for increasing the speed, intensity and persistence of the release of the foreign substances.

This method may be implemented essentially by coating onto the surfaces of the yeast cells that encapsulate foreign substances (foreign-substance encapsulating yeast cells) at least one member selected from the group consisting of saccharides and proteins. The yeast cells used herein are preferably yeasts with their endogenous intracellular components eluted beforehand.

Herein, the yeasts, foreign substances, saccharides, and proteins used in the aforementioned method may be those described in section (1) above, while the methods for eluting the endogenous intracellular component out of the yeast cells, the methods for encapsulating the foreign substances in the yeast cells, and the methods for coating saccharides and proteins onto the surface of the yeast may be carried out also in accordance with the method described in section (1) above. Also, the extent to which the saccharides and proteins ('saccharides, etc.') are coated onto the surface of the yeast cells encapsulating the foreign substances is not particularly restricted, so that the yeast may be completely or partially covered. The 'saccharides, etc.' may also permeate /penetrate into the yeast cell wall or inside the cell, without any particular restriction. The proportion of 'saccharides, etc.' coated on the surface of the yeast cells encapsulating the foreign substances, relative to 100 wt% of the foreign-substance encapsulating yeast usually ranges from 1 to 90 wt%, preferably from 10 to 50 wt%, and yet more preferably from 20 to 40 wt% (dried solids weight ratios).

The microcapsules (microcapsules with coated "saccharides, etc.) thus obtained allow the control of the speed, intensity and persistence of the release of the foreign substances encapsulated therein. This effect becomes more pronounced especially by placing the "microcapsules with coated 'saccharides, etc.'" in the presence of water, in particular by placing them inside the mouth. Therefore, the method according to the present invention may be suitably used for controlling the speed, intensity and persistence of the release of flavor/taste components inside the mouth.

Thus the present invention provides a suitable method for controlling the speed, intensity and persistence of the expression of flavor/taste, preferably for increasing the speed, intensity and persistence of the expression of flavor/taste, in microcapsules of a flavor/taste components encapsulated in yeast cells (foreign-substance encapsulating yeast cells).

Flavor/taste components herein include compounds for building flavor (smell) or taste. These include, but are not limited to, flavorings, sweeteners (including saccharides), acidulants, seasonings, bitterants, spices, fats/lipids, vitamins, amino acids, and animal and plant extracts, etc. More preferably, they include flavorings, sweeteners, acidulants, seasonings, bitterants and spices, yet more preferably flavorings and sweeteners, and more preferably still flavorings. These components are all preferably edible components suitable for use in foods. Yet more preferably, they are hydrophobic components such as hydrophobic flavorings, etc.

The "microcapsules with coated 'saccharides, etc.'" obtained by coating with 'saccharides, etc.' onto the surfaces of yeast cells encapsulating flavor/taste components (microcapsules) are more effective in achieving a faster expression of flavor/taste in the mouth and prolonging a moderate intensity of flavor/taste than is the case for unmodified yeast cells encapsulating a flavor/taste components (microcapsule).

The method according to the present invention is therefore a method for controlling the release of flavor/taste components encapsulated in microcapsules (yeast cells) from the microcapsules. Specifically, the method according to the present invention may be used as a technique for increasing the speed, intensity and persistence of the release or expression of flavor/taste components (flavorings, sweeteners, acidulants, seasonings, bitterants, spices, fats/lipids, vitamins, amino acids, or animal/plant extracts, etc.).

Furthermore, by including as flavor/taste components (flavorings, sweeteners, acidulants, seasonings, bitterants, spices, fats/lipids, vitamins, amino acids, or animal/plant extracts, etc.) the microcapsules according to the present invention used herein (microcapsules with coated 'saccharides, etc.') in oral compositions such as food, etc., it becomes possible to control the speed, intensity and persistence of the expression of flavor/taste in such oral compositions.

Thus the present invention provides a suitable method for controlling the speed, intensity and persistence of the expression of flavor/taste, preferably for increasing the speed, intensity and persistence of the expression of flavor/taste, in oral compositions such as food, etc. Suitable oral compositions herein include compositions held or chewed for a prolonged time in the mouth, for example food compositions such as chewing gum, soft candy, etc.; pharmaceutical or quasi-pharmaceutical compositions such as chewable tablets, masticatories, buccal tablets, dentifrices, etc. A preferred oral composition herein is chewing gum. This method may be implemented by using as a component of the target oral composition the "microcapsules with coated 'saccharides, etc.'" above, i.e., by preparing the target oral composition using the "microcapsules with coated 'saccharides, etc.'" as one component thereof. The proportion of "microcapsules with coated 'saccharides, etc.'" used in the preparation the oral composition, in particular chewing gum, etc. may be those described in section (1) above, while the preparation method thereof may be carried out also in accordance with the method described in section (1) above.

### EXAMPLES

The present invention will be described below in detail with reference to the following Examples, Comparative Examples, and Experiments, but is not limited thereto. In each Example, unless otherwise specified, "part" means "part by weight" and "%" means "% by weight".

### Examples 1 to 6

Saccharide-coated microcapsule containing flavorings (intracellular components removed)

### (Microencapsulated flavoring)

### (1) Removal of endogenous intracellular components from the yeast cell body

2 g of protease was added to 2,000 g of slurry in which 100 g of a brewer's yeast (*saccharomyces cerevisiae*) was dispersed in water, and the resulting mixture was then shaken at 50°C for 20 hours to elute the intracellular components from the interior of the cell outside. The obtained slurry was separated by centrifugation at 8,000 rpm for 30 minutes and the supernatant was removed, giving 350 g (solids content of 20%) of yeast residue. 930 g of water and 120 g of concentrated hydrochloric acid were added thereto and the resulting mixture was heated for 10 minutes at 80°C while stirring (acid treatment), followed by cooling. After centrifuging at 8,000 rpm for 30 minutes, the supernatant was removed, giving 500 g (solids content of 10%) of acid-treated yeast residue. Subsequently, 500 g of water was added thereto, followed by centrifugation at 8,000 rpm for 30 minutes, and the supernatant was removed. 10% aqueous sodium hydroxide was added to 500 g of the obtained residue (solids content of 10%) and the resulting solution was adjusted to pH 7.0.

### (2) Inclusion of flavorings into yeast cell bodies

450 g (solids content of 10%) of the above-prepared aqueous dispersion containing the yeast from which endogenous components had been removed from the yeast cell bodies was heated at 70°C, and 25 g of menthol was added thereto, followed by stirring at 5,000 rpm for 2 hours, whereby a flavoring (menthol) was included into the yeast cell bodies (flavoring-containing yeast).

### (3) Coating of saccharides to the surface of yeast cell bodies

475 g of each of the thus prepared aqueous solutions containing the flavoring-containing yeast were prepared (6 types). To each aqueous solution was mixed either 60 g of an aqueous solution with a lactose content of 50% heated at 60°C (Example 1), 60 g of an aqueous solution with a dextrin (DE=10) content of 50% (Example 2), 60 g of aqueous solution with a maltose content of 50% (Example 3), 60 g of an aqueous solution with reduced paratinose content of 50% (Example 4), 120 g of an aqueous solution with a gelatin content of 25% (Example 5), or 100 g of an aqueous solution containing 15% gelatin and 15% lactose (Example 6). Subsequently, these mixed solutions were spray-dried with a spray dryer at an inlet temperature of 150°C and an outlet temperature of 90°C, to give 90 g each of powdered "saccharide-coated microcapsule containing flavorings (intracellular components removed)" (Examples 1 to 6).
Example 6 is a Reference Example.

### Example 7

Saccharide-coated microcapsule containing flavorings (Intracellular components contained)

### (Microencapsulated flavorings)

A procedure was conducted (using an aqueous solution with a lactose content of 50%) in the same manner as in Example 1 except that a brewer's yeast (*saccharomyces cerevisiae*) containing endogenous intracellular components was used in place of the yeast from which endogenous intracellular components had been removed from the cell body, to give a powdered saccharide-coated microcapsule containing flavorings (intracellular components contained) (Example 7).

450 g of an aqueous solution containing 45 g of yeast (*saccharomyces cerevisiae*) was heated at 70°C according to the method described in Example 1 (2), and 25 g of menthol was added thereto, followed by stirring at 5,000 r.p.m. for 2 hours, whereby a flavoring (menthol) was included in the yeast cell bodies (flavoring-containing yeast). To 475 g of an aqueous solution containing the thus-prepared flavoring-containing yeast was mixed 30 g of an aqueous solution with a lactose content of 50% heated at 60°C according to the method described in the above-described Example 1 (3). Subsequently, the mixed solution was spray-dried with a spray dryer at an inlet temperature of 150°C and an outlet temperature of 90°C, to prepare 90 g of powdered "saccharide-coated microcapsule containing flavorings (intracellular components contained)" (Example 7).

### Examples 8 to 14 Chewing gum

The microcapsule containing flavor prepared in Examples 1 to 7 were used as a flavoring ingredient (microencapsulated flavoring) to prepare chewing gums according to the following formulation. Chewing gums were prepared in the usual manner.

### <Formulation of chewing gum>

| | |
|---|---|
| Gum base | 25 parts |
| Sugar | 62 parts |
| Corn syrup | 10 parts |
| Glycerin | 1 part |
| Microencapsulated flavoring | 2 parts |
| Total | 100 parts |

### Example 15

Saccharide-coated microcapsule containing flavorings (Intracellular components removed)

### (Microencapsulated flavorings)

450 g of a yeast solution (solids content of 10%) was prepared by dispersing in water yeast from which endogenous intracellular components had been removed from the cell body prepared in the same manner as in Example 1 (1). The solution was heated at 70°C, and 25 g of orange oil was added thereto, followed by stirring at 5,000 rpm for 2 hours. 60 g of an aqueous solution with lactose content of 50% was added thereto and mixed. Subsequently, the mixed solution was spray-dried with a spray dryer at an inlet temperature of 150°C and an outlet temperature of 90°C, to obtain 90 g of a powdered "saccharide-coated microcapsule containing flavorings (intracellular components removed)" (Example 15).

### Comparative Example 1 Microcapsule containing flavorings (intracellular components removed)

643 g of a yeast solution (solids content of 10%) was prepared by dispersing in water yeast from which endogenous intracellular components had been removed from the cell body prepared in the same manner as in Example 1 (1). The solution was heated at 70°C, and 36 g of menthol was added thereto, followed by stirring at 5,000 rpm for 2 hours. Subsequently, the solution was spray-dried with a spray dryer at an inlet temperature of 150°C and an outlet temperature of 90°C, to obtain 90 g of a powdered microcapsule (microcapsule containing flavorings (intracellular components removed)) (Comparative Example 1).

### Comparative Example 2 Mixed composition of a microcapsule containing flavorings (intracellular components removed) and lactose

30 g of powdered lactose was added and mixed to 70 g of powdered microcapsules (microcapsule containing flavorings (intracellular components removed)) prepared in Comparative Example 1, giving 98 g of a powdered microcapsule content composite (flavoring-encapsulated microcapsule (intracellular components removed) and lactose) (Comparative Example 2).

### Comparative Example 3 Microcapsule containing flavorings (intracellular components contained)

750 g of a yeast solution (solids content of 10%) was prepared by dispersing 75 g of brewer's yeast (*saccharomyces cerevisiae*) in water. The resulting solution was heated at 70°C, and 25 g of menthol was added thereto, followed by stirring at 5,000 rpm for 2 hours. Subsequently, the solution was spray-dried with a spray dryer at an inlet temperature of 150°C and an outlet temperature of 90°C, to prepare 90 g of powdered microcapsule (microcapsule containing flavorings (intracellular components containing)) (Comparative Example 3).

### Comparative Examples 4 to 6 Chewing gum

Each of the microcapsule examples prepared in Comparative Examples 1 to 3 was used as a flavoring ingredient (microencapsulated flavorings) to prepare chewing gums (Comparative Examples 4 to 6) in the same manner as in the above Examples 8 to 14.

### Comparative Example 7 Microcapsule containing flavorings (intracellular components removed)

750 g of a yeast solution (solids content of 10%) was prepared by dispersing in water yeast from which endogenous intracellular components had been removed from the cell body prepared in the same manner as in Example 1 (1). The solution was heated at 70°C, and 25 g of orange oil was added thereto, followed by stirring at 5,000 rpm for 2 hours. Subsequently, the solution was spray-dried with a spray dryer at an inlet temperature of 150°C and an outlet temperature of 90°C, to prepare 90 g of powdered microcapsule (microcapsule containing flavorings (intracellular components removed)) (Comparative Example 7).

### Comparative Example 8 Microencapsulated flavors (Non-microorganism)

250 g of a solution (solids content of 30%) prepared by dissolving 30 g of gum arabic and 30 g of lactose in water was heated to 50°C, and 25 g of orange oil was added thereto, followed by stirring at 5,000 rpm for 2 hours. Subsequently, the resulting solution was spray-dried with a spray dryer at an inlet temperature of 150°C and an outlet temperature of 90°C, to obtain 90 g of a powdered microencapsulated flavorings.

### Experimental Example 1

Expression speed and persistence of the flavor/taste in the microcapsule containing flavorings (microcapsulated flavorings) prepared in Example 1 and Comparative Example 2 were evaluated. (Example 1: lactose-coated microcapsule containing flavorings (intracellular components removed), Comparative Example 1: microcapsule containing flavor (intracellular components removed) + lactose). Specifically, six expert panelists chewed 75 times per minute the chewing gum (Example 8 and Comparative Example 5) comprising the microcapsulated flavorings (Example 1 and Comparative Example 2) as the flavoring ingredient. The speed of the expression, intensity and persistence of the flavor/taste in the mouse were evaluated compared to those of chewing gum (Comparative Example 4) prepared using the microcapsule (microcapsulated flavorings: flavorings-containing microcapsule (intracellular components removed)) of Comparative Example 1 as a flavoring ingredient, which was defined as the control. The evaluation was conducted based on the criteria shown in Table 1 with the expression speed, intensity and persistence of flavor/taste of the control-chewing gum (Comparative Example 4) being defined as 3.

**Table 1**

| Evaluation scale | Expression speed of the flavor/taste | Intensity of the flavor/taste | Persistence of the flavor/taste |
|---|---|---|---|
| 5 | Fast | Strong | Long |
| 4 | Relatively fast | Relatively strong | Relatively long |
| 3 (Control) | Average | Average | Average |
| 2 | Relatively slow | Relatively weak | Relatively short |
| 1 | Slow | Weak | Short |

Table 2 shows the results. The scores are expressed as the average of those of the six expert panelists.

**Table 2**

| | Types of saccharide | State of saccharide | Expression speed of the flavor/taste | Intensity of the flavor/taste | Persistence of the flavor/taste |
|---|---|---|---|---|---|
| Examples 1/8 | Lactose | Coated | 4.8 | 4.8 | 4.8 |
| Comparative Examples 2/5 | Lactose | Mixed | 3.0 | 3.4 | 3.0 |

In the table, "Examples 1/8" and "Comparative Examples 2/5" respectively indicate Examples 1 and 8, and Comparative Examples 2 and 5. The tables below follow the same rule.

As is clear from the results of the test conducted using the chewing gum of Example 8, it is possible to prepare chewing gum having a fast expression, intense and persistent of flavor/taste inside the mouth by using a microcapsulated flavoring (Example 1) comprising a flavoring-containing yeast (intracellular components removed) covered with lactose as a flavoring ingredient. Chewing gum (Comparative Example 5) prepared by using a microcapsulated flavoring (Comparative Example 2) obtained by merely mixing a flavoring-containing yeast (intracellular components removed) and lactose as a flavoring ingredient was evaluated almost the same as the control gum (speed of expression, intensity and persistence of flavor/taste in the mouth: 3.0) in which the speed of expression, intensity and persistence of flavor/taste are significantly inferior to the gum of Example 8 of the present invention. These results made it clear that by coating to or covering the surface of the flavor-containing yeast with lactose, the distinctive effects of the present invention can be achieved, and these cannot be attained by merely mixing the yeast with lactose.

### Experimental Example 2

Expression speed, intensity and persistence of flavor/taste were evaluated in the same manner as in Experimental Example 1, using chewing gum (Example 12 and Comparative Example 6) prepared using microcapsulated flavorings of Example 7 and Comparative Example 3 (Example 7: lactose-coated microcapsule containing flavorings (intracellular components containing), Comparative Example 3: flavoring-encapsulated microcapsule (intracellular components containing)) as a flavoring ingredient without removing the endogenous intracellular components from yeast cell body. Table 3 shows the results.

**Table 3**

| | Presence of intracellular components | Presence of saccharide | Expression speed of the flavor/taste | Intensity of the flavor/ taste | Persistence of the flavor/taste |
|---|---|---|---|---|---|
| Examples 7/14 | Present | Lactose | 4.8 | 3.6 | 3.6 |
| Comp. Examples 3/6 | Present | Not present | 3.0 | 3.0 | 3.0 |

As is clear from these results, the chewing gum (Example 14) comprising the microcapsulated flavorings (Example 7: lactose-coated microcapsule containing flavorings (intracellular components containing)) that was prepared by enclosing the flavorings in yeast without removing the endogenous intracellular component from the yeast cell body and coating lactose on the surface thereof was significantly superior to chewing gum (Comparative Example 6) comprising the microcapsulated flavorings (Comparative Example 3: flavoring-encapsulated microcapsule (intracellular components containing)) obtained without coating saccharide on the surface of the cell body in the expression speed, intensity and persistence of flavorings.

### Experimental Example 3

Expression (speed and intensity) and persistence of flavor were evaluated in the same manner as in Experimental Example 1 using chewing gum (Examples 9 to 11) prepared in the same manner as Example 8 except that the microcapsulated flavorings obtained in Example 2 to 4 (Example 2: dextrin-coated microcapsule containing flavorings (intracellular components removed), Example 3: maltose-coated microcapsule containing flavorings (intracellular components removed), Example 4: reduced palatinit-coated microcapsule containing flavorings (intracellular components removed)) instead of that obtained in Example 1 (lactose-coated microcapsule containing flavorings (intracellular components removed)).

The results are shown in table 4 together with the test results obtained using the chewing gum (using lactose as the coated saccharide) of Example 8.

**Table 4**

| | Types of the coated saccharide | Expression speed of the flavor/taste | Intensity of the flavor/taste | Persistence of the flavor/taste |
|---|---|---|---|---|
| Examples 1/8 | Lactose | 4.8 | 4.8 | 4.8 |
| Examples 2/9 | Dextrin (DE10) | 4.2 | 3.0 | 4.8 |
| Examples 3/10 | Maltose | 2.4 | 2.8 | 4.8 |
| Examples 4/11 | Reduced palatinit | 5.0 | 4.0 | 3.6 |
| Examples 5/12 | Gelatin | 3.0 | 2.0 | 5.0 |
| Examples 6/13 | Gelatin + Lactose | 4.0 | 2.5 | 4.9 |

As is clear from the results of Examples 9 and 10, the microcapsulated flavorings having the surfaces covered with dextrin (DE10) or maltose exhibited an intensity of the flavor/taste almost the same as that of the control (Comparative Example 4); however, the persistence of flavor/taste was significantly improved. As is clear form the results of Example 11, the microcapsulated flavorings with their surface covered by reduced palatinit remarkably improved the intensity of the flavor/taste.

Furthermore, the results of Examples 12 and 13 show that microcapsulated flavorings having their surfaces covered with gelatin or gelatin + lactose exhibited a flavor intensity weaker than that of the control (Comparative Example 4); however, the persistence of the flavor/taste thereof was significantly improved.

### Experimental Example 4

Microcapsules of Example 15 and Comparative Examples 7 and 8 (Example 15: saccharide-coated microcapsule containing flavorings (intracellular components removed), Comparative Example 7: flavoring-encapsulated microcapsule (intracellular components removed), Comparative Example 8: microcapsulated flavorings (non- microbial)) were evaluated for the intensity and persistence of flavor/tasted.

Specifically, 50 g of each microcapsule was put into a plastic bag, sealed, and stored at 50°C for a month. To evaluate the persistence of flavor/taste enclosed in the microcapsules, the content of limonene (orange flavor) in the microcapsules stored for a month was measured and the remaining ratio thereof was calculated, having defined the content of limonene before storage as 100%. The microcapsules (before storage) of Example 15 and Comparative Examples 7 and 8 contained 23 % limonene based on the weight of the microcapsule (100%).

To examine the change in quality of the flavorings after storage, 0.1% solutions were prepared using the microcapsules before and after storage at 50°C for a month, and six expert panelists evaluated the flavor. Table 5 shows the results.

**Table 5**

| | Remaining amount of limonene | Flavoring of 0.1% solution |
|---|---|---|
| Example 15 | 95% | No change |
| Comp. Example 7 | 70% | Slightly oxidized odor |
| Comp. Example 8 | 45% | Strongly oxidized odor |

From these results, it became clear that persistence of flavor can be improved by covering the surface of the flavoring-encapsulated yeast (microcapsule) with saccharide (Example 15), and changes in quality of the flavor can be significantly prevented.

The present invention can improve the release speed, intensity and persistence of foreign materials contained in yeast cells (microcapsules). The microcapsules having a controlled and/or improved speed, intensity and/or persistence of release of the foreign material can be used as various products, such as foods, pharmaceuticals, quasi- pharmaceuticals, cosmetics and miscellaneous goods depending on the foreign material enclosed therein. For example, an edible flavoring-encapsulated microcapsule of the present invention can be used as a flavoring ingredient in chewing gum. A chewing gum that contains the microcapsule as a flavoring ingredient exhibits the effects of, depending on the type of saccharide and polyhdric alcohols coated, promptly releasing the flavor in the mouth, releasing the flavor intensely in the mouth, and maintaining the flavor/taste for a long time of period.

## Claims

1. Microcapsules;
(a) having at least one foreign substance encapsulated in yeast cells whose surfaces are coated with at least one member selected from the group consisting of monosaccharides, disaccharides, trisaccharides, tetrasaccharides, oligosaccharides, dextrin, gum arabic, xanthan gum, guar gum, carrageenan, curdlan, tamarind seed gum, gellan gum, starches, modified starches and sugar alcohols, wherein the foreign substance encapsulated in the yeast cells is at least one member selected from the group consisting of flavorings, colorings, acidulants, seasonings, bitterants, spices, sweeteners, fats/lipids, fatty acids, amino acids, enzymes, vitamins, animal and plant extracts, and bioactive substances, or
(b) having at least one foreign substance encapsulated in yeast cells whose surfaces are coated with at least one member selected from the group consisting of proteins, wherein the foreign substance encapsulated in the yeast cells is at least one hydrophobic substance selected from the group consisting of flavorings, colorings, acidulants, seasonings, bitterants, spices, sweeteners, fats/lipids, fatty acids, amino acids, enzymes, vitamins, animal and plant extracts, and bioactive substances.

2. The microcapsules according to claim 1, wherein the yeast cells are cells that have been previously treated to remove the endogenous intracellular components.

3. The microcapsules according to claim 1, wherein the surfaces of the yeast cells are coated with at least one member selected from the group consisting of lactose, trehalose, palatinit, dextrin, glucose, maltose, maltooligosaccharides and mannitol.

4. The microcapsules according to claim 1, wherein the foreign substance encapsulated in the yeast cells whose surfaces are coated with at least one member selected from the group consisting of monosaccharides, disaccharides, trisaccharides, tetrasaccharides, oligosaccharides, dextrin, gum arabic, xanthan gum, guar gum, carrageenan, curdlan, tamarind seed gum, gellan gum, starches, modified starches and sugar alcohols is a hydrophobic substance.

5. The microcapsules according to claim 1, wherein the foreign substance encapsulated in the yeast cells whose surfaces are coated with at least one member selected from the group consisting of monosaccharides, disaccharides, trisaccharides, tetrasaccharides, oligosaccharides, dextrin, gum arabic, xanthan gum, guar gum, carrageenan, curdlan, tamarind seed gum, gellan gum, starches, modified starches and sugar alcohols is at least one member selected from the group consisting of flavorings, acidulants, bitterants, spices, sweeteners, fatty acids, and vitamins.

6. The microcapsules according to claim 1, wherein the hydrophobic substance encapsulated in the yeast cells whose surfaces are coated with at least one member selected from the group consisting of proteins is at least one member selected from the group consisting of flavorings, acidulants, bitterants, spices, sweeteners, fatty acids, and vitamins.

7. The microcapsules according to claim 1, which are used as a constituent in foods, pharmaceuticals, quasi-pharmaceuticals, cosmetics or feeds.

8. A manufacturing method of microcapsules comprising the steps of:
(1) encapsulating a foreign substance inside yeast cells, wherein the foreign substance is at least one member selected from the group consisting of flavorings, colorings, acidulants, seasonings, bitterants, spices, sweeteners, fats/lipids, fatty acids, amino acids, enzymes, vitamins, animal and plant extracts, and bioactive substances, and
(2) coating the surface of the yeast cells obtained in step (1) with at least one member selected from the group consisting of monosaccharides, disaccharides, trisaccharides, tetrasaccharides, oligosaccharides, dextrin, gum arabic, xanthan gum, guar gum, carrageenan, curdlan, tamarind seed gum, gellan gum, starches, modified starches and sugar alcohols.

9. A manufacturing method of microcapsules comprising the steps of:
(1) encapsulating a foreign substance inside yeast cells, wherein the foreign substance is at least one hydrophobic substance selected from the group consisting of flavorings, colorings, acidulants, seasonings, bitterants, spices, sweeteners, fats/lipids, fatty acids, amino acids, enzymes, vitamins, animal and plant extracts, and bioactive substances, and
(2) coating the surface of the yeast cells obtained in step (1) with at least one member selected from the group consisting of proteins.

10. The manufacturing method of microcapsules according to claim 8 or 9, wherein step (1) is a step of encapsulating a foreign substance inside yeast cells whose endogenous intracellular components have been removed.

11. An oral composition containing microcapsules of claim 1.

12. The oral composition according to claim 11, which contains the microcapsules of claim 1 as flavorings, colorings, seasonings, acidulants, bitterants, spices or sweeteners.

13. The oral composition according to claim 11, which is held or chewed in the mouth for a prolonged time.

14. The oral composition according to claim 11, which is a chewing gum.

15. A method for controlling the speed, intensity or persistence of the foreign substance released from microcapsules of foreign substances encapsulated in yeast cells, comprising the steps of:
(a) coating the surface of these microcapsules with at least one member selected from the group consisting of monosaccharides, disaccharides, trisaccharides, tetrasaccharides, oligosaccharides, dextrin, gum arabic, xanthan gum, guar gum, carrageenan, curdlan, tamarind seed gum, gellan gum, starches, modified starches and sugar alcohols, wherein the foreign substance is at least one member selected from the group consisting of flavorings, colorings, acidulants, seasonings, bitterants, spices, sweeteners, fats/lipids, fatty acids, amino acids, enzymes, vitamins, animal and plant extracts, and bioactive substances, or
(b) coating the surface of these microcapsules with at least one member selected from the group consisting of proteins, wherein the foreign substance is at least one hydrophobic substance selected from the group consisting of flavorings, colorings, acidulants, seasonings, bitterants, spices, sweeteners, fats/lipids, fatty acids, amino acids, enzymes, vitamins, animal and plant extracts, and bioactive substances.

16. The method according to claim 15, which is a method for controlling the speed, intensity or persistence of the expression of the flavor/taste from microcapsules of a flavor/taste component encapsulated in the yeast cells.

17. The method according to claim 16, wherein the microcapsules of the flavor/taste component encapsulated in the yeast cells are obtained by encapsulating a flavor/taste component in yeast cells whose endogenous intracellular components have been removed.

18. The method according to claim 15, which is a method for increasing the speed, intensity or persistence of the expression of the flavor/taste from the microcapsules.

19. Use of microcapsules having foreign substances encapsulated inside yeast cells;
(a) wherein the surfaces of the yeast cells are coated with at least one member selected from the group consisting of monosaccharides, disaccharides, trisaccharides, tetrasaccharides, oligosaccharides, dextrin, gum arabic, xanthan gum, guar gum, carrageenan, curdlan, tamarind seed gum, gellan gum, starches, modified starches and sugar alcohols, and
wherein the foreign substance is at least one member selected from the group consisting of flavorings, colorings, acidulants, seasonings, bitterants, spices, sweeteners, fats/lipids, fatty acids, amino acids, enzymes, vitamins, animal and plant extracts, and bioactive substances, or
(b) wherein the surfaces of the yeast cells are coated with at least one member selected from the group consisting of proteins, and
wherein the foreign substance is at least one hydrophobic substance selected from the group consisting of flavorings, colorings, acidulants, seasonings, bitterants, spices, sweeteners, fats/lipids, fatty acids, amino acids, enzymes, vitamins, animal and plant extracts, and bioactive substances,
as flavorings, sweeteners, colorings, seasonings, bitterants, spices or acidulants

20. Use of microcapsules having foreign substances encapsulated inside yeast cells for the preparation of an oral composition;
(a) wherein the surfaces of the yeast cells are coated with at least one member selected from the group consisting of monosaccharides, disaccharides, trisaccharides, tetrasaccharides, oligosaccharides, dextrin, gum arabic, xanthan gum, guar gum, carrageenan, curdlan, tamarind seed gum, gellan gum, starches, modified starches and sugar alcohols, and wherein the foreign substance is at least one member selected from the group consisting of flavorings, colorings, acidulants, seasonings, bitterants, spices, sweeteners, fats/lipids, fatty acids, amino acids, enzymes, vitamins, animal and plant extracts, and bioactive substances, or
(b) wherein the surfaces of the yeast cells are coated with at least one member selected from the group consisting of proteins, and wherein the foreign substance is at least one hydrophobic substance selected from the group consisting of flavorings, colorings, acidulants, seasonings, bitterants, spices, sweeteners, fats/lipids, fatty acids, amino acids, enzymes, vitamins, animal and plant extracts, and bioactive substances.

## Patentansprüche

1. Mikrokapseln,
(a) die mindestens eine fremde Substanz verkapselt in Hefezellen aufweisen, deren Oberflächen mit mindestens einem Mitglied, ausgewählt aus der Gruppe, bestehend aus Monosacchariden, Disacchariden, Trisacchariden, Tetrasacchariden, Oligosacchariden, Dextrin, Gummi arabicum, Xanthangummi, Guargummi, Carrageen, Curdlan, Tamarindensamengummi, Gellangummi, Stärken, modifizierten Stärken und Zuckeralkoholen, beschichtet sind, wobei die in die Hefezellen verkapselte fremde Substanz mindestens ein Mitglied, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen ist, oder
(b) die mindestens eine fremde Substanz verkapselt in Hefezellen aufweisen, deren Oberflächen mit mindestens einem Mitglied, ausgewählt aus der Gruppe, bestehend aus Proteinen, beschichtet sind, wobei die in die Hefezellen verkapselte fremde Substanz mindestens ein Mitglied, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen ist.

2. Mikrokapseln nach Anspruch 1, wobei die Hefezellen Zellen sind, die zuvor behandelt wurden, um die endogenen intrazellulären Bestandteile zu entfernen.

3. Mikrokapseln nach Anspruch 1, wobei die Oberflächen der Hefezellen mit mindestens einem Mitglied, ausgewählt aus der Gruppe, bestehend aus Lactose, Maltitose, Trehalose, Palatinit, Dextrin, Glucose, Maltose, Maltooligosacchariden und Mannitol, beschichtet sind.

4. Mikrokapseln nach Anspruch 1, wobei die in die Hefezellen verkapselte fremde Substanz, deren Oberflächen mit mindestens einem Mitglied, ausgewählt aus der Gruppe, bestehend aus Monosacchariden, Disacchariden, Trisacchariden, Tetrasacchariden, Oligosacchariden, Dextrin, Gummi arabicum, Xanthangummi, Guargummi, Carrageen, Curdlan, Tamarindensamengummi, Gellangummi, Stärken, modifizierten Stärken und Zuckeralkoholen, beschichtet sind, eine hydrophobe Substanz ist.

5. Mikrokapseln nach Anspruch 1, wobei die in die Hefezellen verkapselte fremde Substanz, deren Oberflächen mit mindestens einem Mitglied, ausgewählt aus der Gruppe, bestehend aus Monosacchariden, Disacchariden, Trisacchariden, Tetrasacchariden, Oligosacchariden, Dextrin, Gummi arabicum, Xanthangummi, Guargummi, Carrageen, Curdlan, Tamarindensamengummi, Gellangummi, Stärken, modifizierten Stärken und Zuckeralkoholen, beschichtet sind, mindestens ein Mitglied, ausgewählt aus der Gruppe, bestehend aus Aromastoffen, Säuerungsmitteln, Bitterstoffen, Gewürzen, Süßstoffen, Fettsäuren und Vitaminen ist.

6. Mikrokapseln nach Anspruch 1, wobei die in die Hefezellen verkapselte hydrophobe Substanz, deren Oberflächen mit mindestens einem Mitglied, ausgewählt aus der Gruppe, bestehend aus Proteinen, beschichtet sind, mindestens ein Mitglied, ausgewählt aus der Gruppe, bestehend aus Aromastoffen, Säuerungsmitteln, Bitterstoffen, Gewürzen, Süßstoffen, Fettsäuren und Vitaminen ist.

7. Mikrokapseln nach Anspruch 1, die als Bestandteil in Nahrungsmitteln, Pharmazeutika, Quasi-Pharmazeutika, Kosmetika oder Futtermitteln verwendet werden.

8. Herstellungsverfahren für Mikrokapseln, umfassend die Schritte:
(1) Verkapseln einer fremden Substanz im Inneren von Hefezellen, wobei die fremde Substanz mindestens ein Mitglied, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen, ist, und
(2) Beschichten der Oberfläche der in Schritt (1) erhaltenen Hefezellen mit mindestens einem Mitglied, ausgewählt aus der Gruppe, bestehend aus Monosacchariden, Disacchariden, Trisacchariden, Tetrasacchariden, Oligosacchariden, Dextrin, Gummi arabicum, Xanthangummi, Guargummi, Carrageen, Curdlan, Tamarindensamengummi, Gellangummi, Stärken, modifizierten Stärken und Zuckeralkoholen.

9. Herstellungsverfahren für Mikrokapseln, umfassend die Schritte:
(1) Verkapseln einer fremden Substanz im Inneren von Hefezellen, wobei die fremde Substanz mindestens eine hydrophobe Substanz, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen, ist, und
(2) Beschichten der Oberfläche der in Schritt (1) erhaltenen Hefezellen mit mindestens einem Mitglied, ausgewählt aus der Gruppe, bestehend aus Proteinen.

10. Herstellungsverfahren für Mikrokapseln nach Anspruch 8, wobei Schritt (1) ein Schritt des Verkapselns einer fremden Substanz im Inneren von Hefezellen ist, deren endogene intrazelluläre Bestandteile entfernt wurden.

11. Orale Zusammensetzung, enthaltend Mikrokapseln nach Anspruch 1.

12. Orale Zusammensetzung nach Anspruch 11, welche die Mikrokapseln nach Anspruch 1 als Aromastoffe, Farbstoffe, Würzmittel, Säuerungsmittel, Bitterstoffe, Gewürze oder Süßstoffe enthält.

13. Orale Zusammensetzung nach Anspruch 11, die für anhaltende Zeit im Mund gehalten oder gekaut wird.

14. Orale Zusammensetzung nach Anspruch 10, die ein Kaugummi ist.

15. Verfahren zum Kontrollieren der Geschwindigkeit, Intensität oder Persistenz der fremden Substanz, die von Mikrokapseln von in Hefezellen verkapselten fremden Substanzen freigesetzt wird, umfassend die Schritte:
(a) Beschichten der Oberfläche dieser Mikrokapseln mit mindestens einem Mitglied, ausgewählt aus der Gruppe, bestehend aus Monosacchariden, Disacchariden, Trisacchariden, Tetrasacchariden, Oligosacchariden, Dextrin, Gummi arabicum, Xanthangummi, Guargummi, Carrageen, Curdlan, Tamarindensamengummi, Gellangummi, Stärken, modifizierten Stärken und Zuckeralkoholen, wobei die fremde Substanz mindestens ein Mitglied, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen ist, oder
(b) Beschichten der Oberfläche dieser Mikrokapseln mit mindestens einem Mitglied, ausgewählt aus der Gruppe, bestehend aus Proteinen, wobei die fremde Substanz mindestens eine hydrophobe Substanz, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen ist.

16. Verfahren nach Anspruch 15, das ein Verfahren zum Kontrollieren der Geschwindigkeit, Intensität oder Persistenz der Expression des Aromas/Geschmacks von Mikrokapseln von einem in Hefezellen verkapselten Aroma-/Geschmacksbestandteil ist.

17. Verfahren nach Anspruch 16, wobei die Mikrokapseln von dem in Hefezellen verkapselten Aroma-/Geschmacksbestandteil durch Verkapseln eines Aroma-/ Geschmacksbestandteils in Hefezellen, deren endogene intrazelluläre Bestandteile entfernt wurden, erhalten werden.

18. Verfahren nach Anspruch 15, das ein Verfahren zum Steigern der Geschwindigkeit, Intensität oder Persistenz der Expression des Aromas/Geschmacks von den Mikrokapseln ist.

19. Verwendung von Mikrokapseln, die im Inneren von Hefezellen verkapselte fremde Substanzen aufweisen,
(a) wobei die Oberflächen der Hefezellen mit mindestens einem Mitglied, ausgewählt aus der Gruppe, bestehend aus Monosacchariden, Disacchariden, Trisacchariden, Tetrasacchariden, Oligosacchariden, Dextrin, Gummi arabicum, Xanthangummi, Guargummi, Carrageen, Curdlan, Tamarindensamengummi, Gellangummi, Stärken, modifizierten Stärken und Zuckeralkoholen, beschichtet sind, und
wobei die fremde Substanz mindestens ein Mitglied, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen, ist, oder
(b) wobei die Oberflächen der Hefezellen mit mindestens einem Mitglied, ausgewählt aus der Gruppe, bestehend aus Proteinen, beschichtet sind, und
wobei die fremde Substanz mindestens eine hydrophobe Substanz, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen, ist,
als Aromastoffe, Süßstoffe, Farbstoffe, Würzmittel, Bitterstoffe, Gewürze oder Säuerungsmittel.

20. Verwendung von Mikrokapseln, die im Inneren von Hefezellen verkapselte fremde Substanzen aufweisen, für die Herstellung einer oralen Zusammensetzung,
(a) wobei die Oberflächen der Hefezellen mit mindestens einem Mitglied, ausgewählt aus der Gruppe, bestehend aus Monosacchariden, Disacchariden, Trisacchariden, Tetrasacchariden, Oligosacchariden, Dextrin, Gummi arabicum, Xanthangummi, Guargummi, Carrageen, Curdlan, Tamarindensamengummi, Gellangummi, Stärken, modifizierten Stärken und Zuckeralkoholen, beschichtet sind, und
wobei die fremde Substanz mindestens ein Mitglied, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen, ist, oder
(b) wobei die Oberflächen der Hefezellen mit mindestens einem Mitglied, ausgewählt aus der Gruppe, bestehend aus Proteinen, beschichtet sind, und
wobei die fremde Substanz mindestens eine hydrophobe Substanz, ausgewählt aus der Gruppe, bestehend aus Aromastoffen, Farbstoffen, Säuerungsmitteln, Würzmitteln, Bitterstoffen, Gewürzen, Süßstoffen, Fetten/Lipiden, Fettsäuren, Aminosäuren, Enzymen, Vitaminen, Tier- und Pflanzenextrakten und bioaktiven Substanzen, ist.

## Revendications

1. Microcapsules ;
(a) ayant au moins une substance étrangère encapsulée dans des cellules de levure dont des surfaces sont recouvertes d'au moins un élément choisi parmi le groupe constitué de monosaccharides, disaccharides, trisaccharides, tétrasaccharides, oligosaccharides, dextrine, gomme arabique, gomme xanthane, gomme de guar, carraghénane, curdlan, gomme de graines de tamarin, gomme gellane, amidons, amidons modifiés et alcools de sucre, dans lesquelles la substance étrangère encapsulée dans les cellules de levure est au moins un élément choisi parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives, ou
(b) ayant au moins une substance étrangère encapsulée dans des cellules de levure dont des surfaces sont recouvertes d'au moins un élément choisi parmi le groupe constitué de protéines, dans lesquelles la substance étrangère encapsulée dans les cellules de levure est au moins une substance hydrophobe choisie parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives.

2. Microcapsules selon la revendication 1, dans lesquelles les cellules de levure sont des cellules qui ont été préalablement traitées pour retirer les composants intracellulaires endogènes.

3. Microcapsules selon la revendication 1, dans lesquelles les surfaces des cellules de levure sont recouvertes d'au moins un élément choisi parmi le groupe constitué de lactose, tréhalose, palatinit, dextrine, glucose, maltose, maltooligosaccharides et mannitol.

4. Microcapsules selon la revendication 1, dans lesquelles la substance étrangère encapsulée dans les cellules de levure dont des surfaces sont recouvertes d'au moins un élément choisi parmi le groupe constitué de monosaccharides, disaccharides, trisaccharides, tétrasaccharides, oligosaccharides, dextrine, gomme arabique, gomme xanthane, gomme de guar, carraghénane, curdlan, gomme de graines de tamarin, gomme gellane, amidons, amidons modifiés et alcools de sucre est une substance hydrophobe.

5. Microcapsules selon la revendication 1, dans lesquelles la substance étrangère encapsulée dans les cellules de levure dont des surfaces sont recouvertes d'au moins un élément choisi parmi le groupe constitué de monosaccharides, disaccharides, trisaccharides, tétrasaccharides, oligosaccharides, dextrine, gomme arabique, gomme xanthane, gomme de guar, carraghénane, curdlan, gomme de graines de tamarin, gomme gellane, amidons, amidons modifiés et alcools de sucre est au moins un élément choisi parmi le groupe constitué d'arômes, acidulants, agents d'amertume, épices, édulcorants, acides gras, et vitamines.

6. Microcapsules selon la revendication 1, dans lesquelles la substance hydrophobe encapsulée dans les cellules de levure dont des surfaces sont recouvertes d'au moins un élément choisi parmi le groupe constitué de protéines est au moins un élément choisi parmi le groupe constitué d'arômes, acidulants, agents d'amertume, épices, édulcorants, acides gras, et vitamines.

7. Microcapsules selon la revendication 1, qui sont utilisées comme constituant dans des aliments, des produits pharmaceutiques, des produits quasi-pharmaceutiques, des cosmétiques ou des aliments pour animaux.

8. Procédé de fabrication de microcapsules comprenant les étapes consistant à :
(1) encapsuler une substance étrangère à l'intérieur de cellules de levure, dans lequel la substance étrangère est au moins un élément choisi parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives, et
(2) revêtir la surface des cellules de levure obtenues à l'étape (1) avec au moins un élément choisi parmi le groupe constitué de monosaccharides, disaccharides, trisaccharides, tétrasaccharides, oligosaccharides, dextrine, gomme arabique, gomme xanthane, gomme de guar, carraghénane, curdlan, gomme de graines de tamarin, gomme gellane, amidons, amidons modifiés et alcools de sucre.

9. Procédé de fabrication de microcapsules comprenant les étapes consistant à :
(1) encapsuler une substance étrangère à l'intérieur de cellules de levure, dans lequel la substance étrangère est au moins une substance hydrophobe choisie parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives, et
(2) revêtir la surface des cellules de levure obtenues à l'étape (1) avec au moins un élément choisi parmi le groupe constitué de protéines.

10. Procédé de fabrication de microcapsules selon la revendication 8 ou 9, dans lequel l'étape (1) est une étape d'encapsulation d'une substance étrangère à l'intérieur de cellules de levure dont des composants intracellulaires endogènes ont été retirés.

11. Composition orale contenant des microcapsules selon la revendication 1.

12. Composition orale selon la revendication 11, qui contient les microcapsules selon la revendication 1 en tant qu'arômes, colorants, assaisonnements, acidulants, agents d'amertume, épices ou édulcorants.

13. Composition orale selon la revendication 11, qui est conservée ou mâchée dans la bouche pendant un temps prolongé.

14. Composition orale selon la revendication 11, qui est un chewinggum.

15. Procédé pour commander la vitesse, l'intensité ou la persistance de la substance étrangère libérée par des microcapsules de substances étrangères encapsulées dans des cellules de levure, comprenant les étapes consistant à :
(a) revêtir la surface de ces microcapsules avec au moins un élément choisi parmi le groupe constitué de monosaccharides, disaccharides, trisaccharides, tétrasaccharides, oligosaccharides, dextrine, gomme arabique, gomme xanthane, gomme de guar, carraghénane, curdlan, gomme de graines de tamarin, gomme gellane, amidons, amidons modifiés et alcools de sucre, dans lequel la substance étrangère est au moins un élément choisi parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives, ou
(b) revêtir la surface de ces microcapsules avec au moins un élément choisi parmi le groupe constitué de protéines, dans lequel la substance étrangère est au moins une substance hydrophobe choisie parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives.

16. Procédé selon la revendication 15, qui est un procédé pour commander la vitesse, l'intensité ou la persistance de l'expression de l'arôme/goût à partir de microcapsules d'un composant d'arôme/goût encapsulé dans les cellules de levure.

17. Procédé selon la revendication 16, dans lequel les microcapsules du composant d'arôme/goût encapsulé dans les cellules de levure sont obtenues en encapsulant un composant d'arôme/goût dans des cellules de levure dont des composants intracellulaires endogènes ont été retirés.

18. Procédé selon la revendication 15, qui est un procédé pour augmenter la vitesse, l'intensité ou la persistance de l'expression de l'arôme/goût à partir des microcapsules.

19. Utilisation de microcapsules ayant des substances étrangères encapsulées à l'intérieur de cellules de levure ;
(a) dans laquelle les surfaces des cellules de levure sont recouvertes d'au moins un élément choisi parmi le groupe constitué de monosaccharides, disaccharides, trisaccharides, tétrasaccharides, oligosaccharides, dextrine, gomme arabique, gomme xanthane, gomme de guar, carraghénane, curdlan, gomme de graines de tamarin, gomme gellane, amidons, amidons modifiés et alcools de sucre, et
dans laquelle la substance étrangère est au moins un élément choisi parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives, ou
(b) dans laquelle les surfaces des cellules de levure sont recouvertes d'au moins un élément choisi parmi le groupe constitué de protéines, et
dans laquelle la substance étrangère est au moins une substance hydrophobe choisie parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives,
comme arômes, édulcorants, colorants, assaisonnements, agents d'amertume, épices ou acidulants.

20. Utilisation de microcapsules ayant des substances étrangères encapsulées à l'intérieur de cellules de levure pour la préparation d'une composition orale ;
(a) dans laquelle les surfaces des cellules de levure sont recouvertes d'au moins un élément choisi parmi le groupe constitué de monosaccharides, disaccharides, trisaccharides, tétrasaccharides, oligosaccharides, dextrine, gomme arabique, gomme xanthane, gomme de guar, carraghénane, curdlan, gomme de graines de tamarin, gomme gellane, amidons, amidons modifiés et alcools de sucre, et dans laquelle la substance étrangère est au moins un élément choisi parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives, ou
(b) dans laquelle les surfaces des cellules de levure sont recouvertes d'au moins un élément choisi parmi le groupe constitué de protéines, et dans laquelle la substance étrangère est au moins une substance hydrophobe sélectionnée parmi le groupe constitué d'arômes, colorants, acidulants, assaisonnements, agents d'amertume, épices, édulcorants, graisses/lipides, acides gras, acides aminés, enzymes, vitamines, extraits d'animal et de plante, et substances bioactives.
